# EUROPEAN PATENT APPLICATION

(11) **EP 3 671 530 A1**
(43) Date of publication of application: **24.06.2020**
(21) Application number: 18212984.1
(22) Date of filing: 17.12.2018
(51) Int. Cl.: G06K 9/00, A61B 5/00

(54) **DEVICE, SYSTEM AND METHOD FOR OBJECT RECOGNITION**

(71) Applicant: Koninklijke Philips N.V., 5656 AG Eindhoven (NL)
(72) Inventor: FERNANDO, Shakith Devinda, 5656 AE Eindhoven (NL); ZHANG, Lu, 5656 AE Eindhoven (NL); VAN DER HEIDE, Esther Marjan, 5656 AE Eindhoven (NL); FALCK, Thomas Maria, 5656 AE Eindhoven (NL)
(74) Representative: de Haan, Poul Erik

(57) **Abstract**

The present invention relates to a device, system and method for object recognition. To improve reliability and robustness of the recognition, the device comprises an input unit (21) configured to obtain a depth image (40) of a scene, a computation unit (22) that computes, from the depth image, a noise variance map (42) by computing pixel noise variances at object boundaries of one or more objects in the depth image, a depth confidence map (43) by filtering depth values based on their distance to the depth camera, and a motion confidence map (44) by filtering out variances caused by motion of a person in the scene. Further, from the noise variance map, the depth confidence map and the motion confidence map, one or more candidate regions (45) and their confidence in the depth image are computed, and the one or more candidate regions having the highest confidence are selected as final region of interest (41) representing the object to be recognized.

## Description

### FIELD OF THE INVENTION

The present invention relates to a device, system and method for object recognition, particularly of a predetermined object. The present invention may e.g. be applied for recognition (or detection) of a bed or other stationary object in a scene, like a chair, cupboard, table, couch, etc., optionally including segmentation and/or localization of the object.

### BACKGROUND OF THE INVENTION

Video monitoring is a popular solution for automatic and remote monitoring in hospitals. A camera system can be placed in patient rooms (e.g., ICUs, general wards, emergency rooms, waiting rooms) to observe and analyze different features (e.g., motion, heart rate, respiration rate) of the patient. This enables diverse applications like delirium monitoring, video-based actigraphy, sleep monitoring, vital signs monitoring. However, such video monitoring is challenging when there are other people beside the patient (e.g., nurses, visitors) in the camera view.

In applications like video-based actigraphy for delirium detection robustness is, however, challenging when there are other people in the camera view besides the patient. In a typical patient room, many activities are completed by the nurse standing very close to the bed. Example activities can be the nurse attaching a breathing tube or changing of patient and bedsheets by a nurse. Furthermore, family members are commonly seen very close to the patient comforting them. Video-based actigraphy becomes an issue when the camera view is occluded by foreground objects (e.g., nurse, family members). Therefore, the key challenge is detecting the patient's region of activity (e.g. bed or chair) when there is partial occlusion from the foreground objects (e.g. a nurse or guest).

JP 2013-078433 A discloses a monitoring device allowing accurate and reproducible detection of movement of a person that is a monitoring target by automatically detecting an area to be monitored with a bed as a reference. A range imaging sensor generates a range image wherein a pixel value is a range value to an object. A visual field area of the range image sensor includes the entirety of the bed that is a monitoring target. A bed recognition unit uses the range image outputted by the range image sensor to extract a position of the bed. Within the range image outputted by the range image sensor, a person recognition unit detects areas occupied by the person inside and outside a range of the bed recognized by the bed recognition unit. A movement decision unit distinguishes the movement of the person to the bed by a combination between the area of the bed detected by the bed recognition unit and the area of the person detected by the person recognition unit.

There is a need for a more reliable and robust detection of objects, e.g. of objects occluding a patient in patient monitoring.

### SUMMARY OF THE INVENTION

It is an object of the present invention to provide a device, system and method for object recognition in a reliable and robust way.

In a first aspect of the present invention a device for object recognition is presented comprising
- an input unit configured to obtain a depth image of a scene, the depth image comprising depth information representing a distance between a depth camera and elements of the scene depicted in the depth image,
- a computation unit configured:
   to compute, from the depth image,
      - a noise variance map by computing pixel noise variances at object boundaries of one or more objects in the depth image,
      - a depth confidence map by filtering depth values based on their distance to the depth camera, and
      - a motion confidence map by filtering out variances caused by motion of a person in the scene,
   to compute, from the noise variance map, the depth confidence map and the motion confidence map, one or more candidate regions and their confidence in the depth image, a candidate region being a region potentially representing an object or a part of the object, and
   to select the one or more candidate regions having the highest confidence as final region of interest representing the object to be recognized.

In a further aspect of the present invention a system for object recognition is presented comprising
- a depth camera configured to acquire a depth image of a scene, the depth image comprising depth information representing a distance between the depth camera and elements of the scene depicted in the depth image,
- a device as disclosed herein for object recognition based on the acquired depth image.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

With known solutions, when there is partial occlusion from a nurse, either the bed region is incorrectly reported or no result is reported at all during the occlusion period. The herewith presented solution allows the detection of only the patient's region of interest (ROI) even with partial occlusion from any object or person. In the application of video-based actigraphy, the presented solution allows the detection of only the motion of interest of the patient even with the co-occurring motion from a person (e.g. when a nurse is attaching a breathing tube while the patient is moving his legs restlessly).

The present invention uses a camera based solution to automatically detect the (particularly predetermined / predefined) object, e.g. a bed border region, while the device disclosed in JP 2013- 078433 A uses physical markers to detect bed borders. Further, the present invention uses a depth image of a scene, e.g. from a depth camera (time of flight camera), to detect variations at object boundaries (e.g. bed borders) and/or at occluding object boundaries (e.g. nurses) to remove objects. Further, combining a noise map with a depth map and a motion confidence map provides for effective contour detection and selecting the best final region. Still further, the object may be segmented and/or localized by use of the present invention.

In an embodiment the computation unit is configured to recognize a bed as the object to be recognized. This is of particularly importance in patient monitoring applications where the patient is lying in a bed. Other objects may be recognized as well in the same or other applications.

There are different options to compute the noise variance map. In one embodiment the computation unit is configured to compute the noise variance map by computing pixel noise variances at boundaries of the object to be recognized and of one or more other objects occluding one or more parts of the object to be recognized in the depth image. In another embodiment the computation unit is configured to compute the noise variance map by use of a noise model that models one or more noise factors. Hereby, the computation unit may be configured to compute the noise variance map (including but not limited to the noise cause e.g. by beds, patients and nurses) by use of a noise model, in particular a Gaussian noise model, that models at least one noise factor selected from a group of noise factors including absorptivity or reflectivity of the material of an object, reflections of light from different objects reaching the same pixel, temporal variations (captured by multiple depth images (a time series) over a time window) depending on when a reflected light reaches the same pixel over time, and one or more pixels having a zero pixel value when no light reaches a pixel or light that would reach a pixel is compensated by other light.

The depth confidence map may be computed by filtering out depth values of pixels lying outside a depth range assigned to the object to be recognized. For instance, an adaptive filter may be applied that adaptively changes the depth range applied for filtering. In another embodiment an object model may be used, in particular a Gaussian object model, which models the depth of the object to be recognized.

The motion confidence map may be computed by using the time duration to induce pixel variations to differentiate between pixel variations caused by motion and pixel variations caused by noise. For instance, the motion confidence map may be computed by looking at multiple depth images over a time window. This time window is preferably larger than the time window for computing the noise variance map. Motion induced variations can then be captured by such a large time window.

In another embodiment the computation unit is configured to compute the one or more candidate regions by computing a joint confidence map from the noise variance map, the depth confidence map and the motion confidence map and to apply contour detection on the joint confidence map to detect contours in the depth image, said contours indicating the one or more candidate regions. Candidate regions may be the regions inside contours and/or a set of contours, wherein each contour may be considered as a candidate region. For instance, for every pixel in frame, it is selected to be part of a contour if all confidence maps for that pixel location indicate it to be relevant as a contour. A pixel located inside a contour will be a part of the corresponding candidate region.

The computation unit may hereby be configured to compute the confidence of the one or more candidate regions by use of a Gaussian distribution on the respective candidate region and multiplying it by the joint confidence map to obtain a region confidence map, and to select the one or more candidate regions having the highest confidence in the joint confidence map as final region of interest representing the object to be recognized.

The computation unit may further be configured to
- rank the one or more candidate regions according to their confidence,
- iteratively combine candidate regions according to their rank,
- compute the sum of their confidence at every iteration,
- stop the iteration when the computed sum of the confidence converges, and
- select the candidate regions combined up to stop of the iteration as final region of interest representing the object to be recognized.

In addition to the device and the depth camera, the system according to the present invention may further comprise an infrared illumination unit configured to illuminate the scene with infrared light, wherein the depth camera is configured to acquire the depth image in the infrared wavelength range.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a system according to the present invention,
Fig. 2 shows a schematic diagram of a first embodiment of a device according to the present invention,
Fig. 3 shows flow chart of an embodiment of a method according to the present invention,
Fig. 4 shows a schematic diagram of a second embodiment of a system and device according to the present invention,
Fig. 5 shows an exemplary depth image,
Fig. 6 shows an exemplary motion confidence map,
Fig. 7 shows an exemplary depth confidence map,
Fig. 8 shows an exemplary noise variance map,
Fig. 9 shows an exemplary region confidence map, and
Fig. 10 shows an exemplary detected region of interest.

### DETAILED DESCRIPTION OF EMBODIMENTS

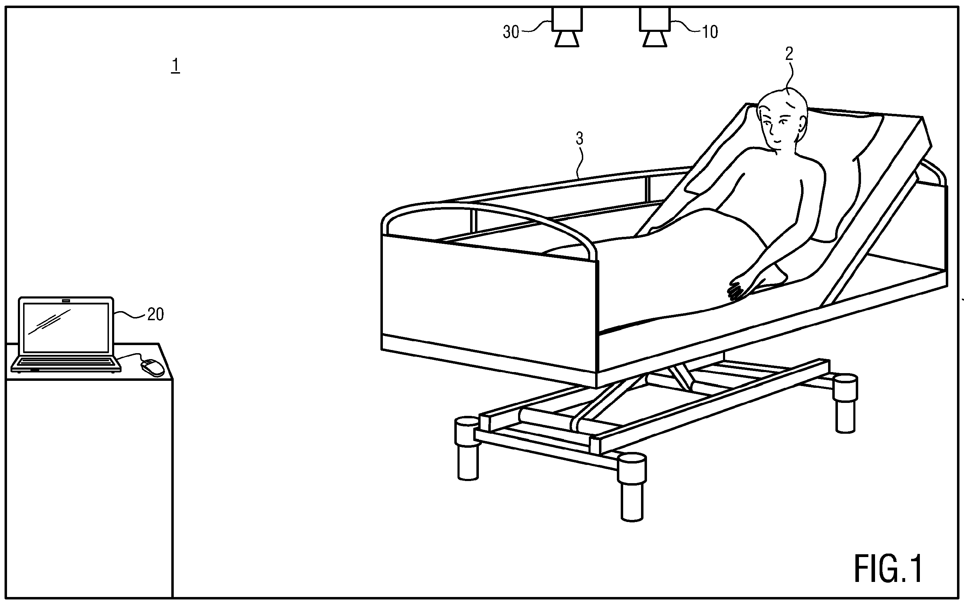

Fig. 1 shows a schematic diagram of an embodiment of a system 1 for objection recognition according to the present invention used in an application for patient monitoring. In the exemplary scenario illustrated in Fig. 1 a patient 2 is lying in a bed 3, e.g. in a hospital room, a room in a care home or at home.

The system 1 comprises a depth camera 10 (also called depth sensor or including a depth sensor, such as a 3D depth camera) that acquire a depth image of the scene. The depth image comprises depth information representing the distance between the depth camera 10 and elements of the scene depicted in the depth image, such as elements of the bed (such as the front plate, the blanket, the inclined head rest, etc.) and visible body parts of the patient (such as the head, the torso, the arms). Preferably, multiple depth images are acquired over time, i.e. a time sequence of depth images, preferably for processing in real time. The time sequence may e.g. be a stream of a number of depth images taken continuously or at regular intervals (e.g. every second, every 5 seconds, every 100 milliseconds, etc.).

The system 1 further comprises a device 20 as disclosed herein and described in more detail below, which uses the acquired depth image for object recognition, i.e. to detect the bed 3 in the scenario shown in Fig. 1 or, in other scenarios, other stationary objects like a chair, a cupboard, a table, a couch, etc. The acquired depth image(s) is (are) preferably provided live (on the fly) and directly provided from camera 10 to the device 20.

The device 20 may generally be implemented in hard- and/or software, e.g. a computer program running on a PC or workstation, as shown in Fig. 1. The device 20 may alternatively be implemented as a processor that is integrated into the camera 10 or any other user device, such as a healthcare provider's (e.g. a nurse's or doctor's) smartphone or table or other equipment carried along or used otherwise by a healthcare provider. The device 20 may thus be mobile or may be stationary, e.g. arranged in the patient's room or in a central monitoring room, such as a nurse's station.

The system 1 may optionally further comprise an infrared (IR) illumination unit 30, such as an IR light source (e.g. an array IR LEDs, preferably near-infrared LEDs) configured to illuminate the scene with infrared light. This is particularly useful, if the depth camera 10 is configured to acquire the depth image in the infrared wavelength range.

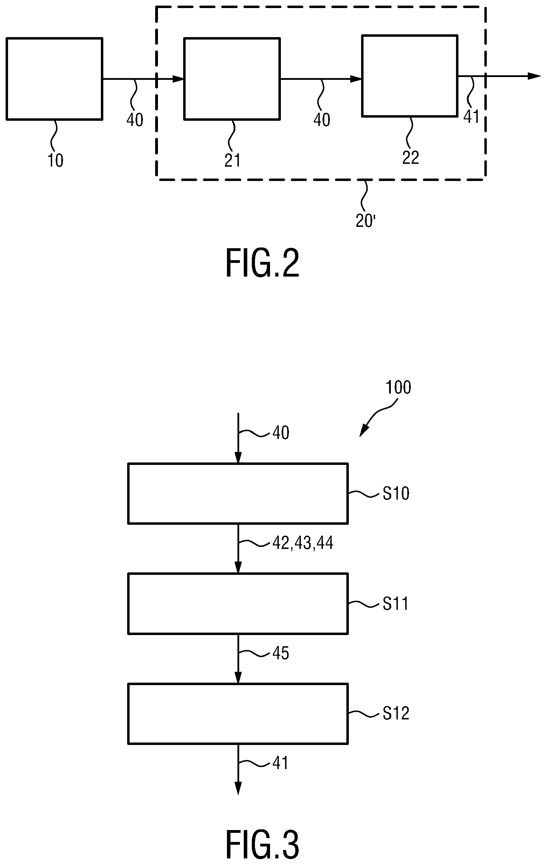

A more detailed embodiment of the device 20, which may be used in the system 1, is schematically depicted in Fig. 2. The device 20 comprises an input unit 21 configured to obtain (i.e. receive or retrieve) a depth image 40 of the scene. The input unit 21 may e.g. be a (wireless or wired) data interface, such as a HDMI, Bluetooth, Wi-Fi or LAN interface that is preferably able to directly obtain depth image(s) 40 from the camera 10.

The device 20 further comprises a computation unit 22 configured to process the obtained depth image(s) by carrying out a number of processing steps in order to recognize the object, in particular to detect a final region of interest representing the object to be recognized. The steps carried out by the computation unit 22 are illustrated in more detail in the flow chart shown in Fig. 3.

In a first step S10 the following three parameters (different maps) for determining the region of interest are computed from the depth image 41: a noise variance map 42, a depth confidence map 43 and a motion confidence map 44. The noise variance map 42 is computed by computing pixel noise variances at object boundaries of one or more objects in the depth image, in particular in order to identify the pixel black holes and pixel noise variances at object boundaries (e.g. nurse, bed borders). The depth confidence map 43 is computed by filtering depth values based on their distance to the depth camera, in particular in order to filter the depth values based on the height from the depth camera. The motion confidence map 44 is computed by filtering out variances caused by motion of a person in the scene, in particular in order to filter out variances due to the motion of the patient in the object region, in the scenario shown in Fig. 1 in the bed region.

In a second step S11, from the noise variance map 42, the depth confidence map 43 and the motion confidence map44, one or more candidate regions 45 and their confidence in the depth image are computed. Hereby, a candidate region is a region potentially representing the object or a part of the object to be recognized.

In a third step S12, the one (or more) candidate region(s) having the highest confidence is (are) selected as final region of interest 41 representing the object to be recognized.

Hence, according to the present invention depth noise variances at object boundaries are exploited for object (e.g. bed) border detection and the similar depth noise variances of occluding object boundaries are exploited for removing occluding objects. The exploited variance feature with the depth confidence map and the motion confidence map are further exploited to further enhance the object boundaries. Finally, contour regions and their confidences are computed from these 3 confidence maps, which are used to find the final region of interest.

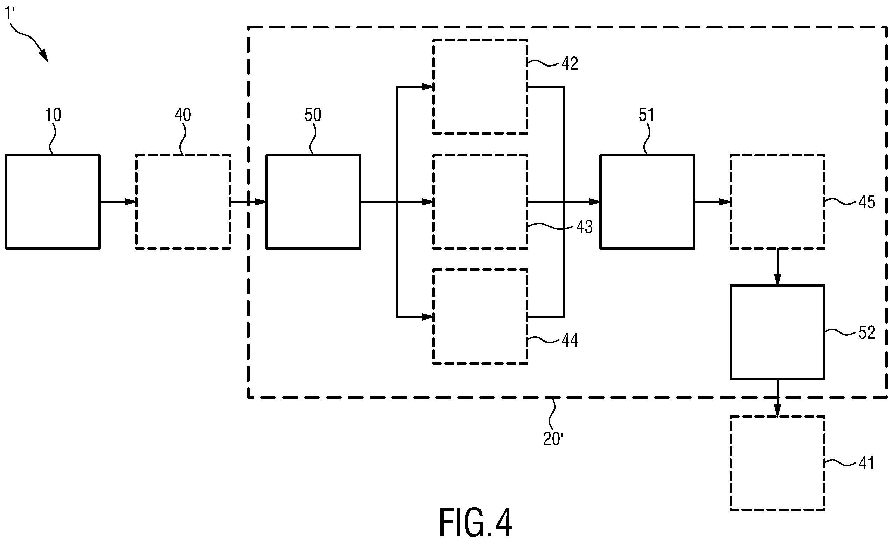

Fig. 4 shows a schematic diagram of a second embodiment of a system 1' and device 20' according to the present invention. In this embodiment the device 20' comprises dedicated units (e.g. software units of a computer program or hardware units of corresponding hardware or circuitry) for performing the steps of the method 100. In particular, an edge pixel confidence computation unit 50 obtains the depth image(s) and carries out step S10 to compute the noise variance map 42, the depth confidence map 43 and the motion confidence map 44. A region confidence computing unit 51 carries out step S11 to compute a region confidence map, i.e. to compute one or more candidate regions 45 and their confidence, also here referred to as a region confidence map 45. A region selection unit 52 carries out step S12 to compute the final region of interest 41 representing the object to be recognized.

In the following the various elements of a practical implementation of the present invention shall be explained in more detail.

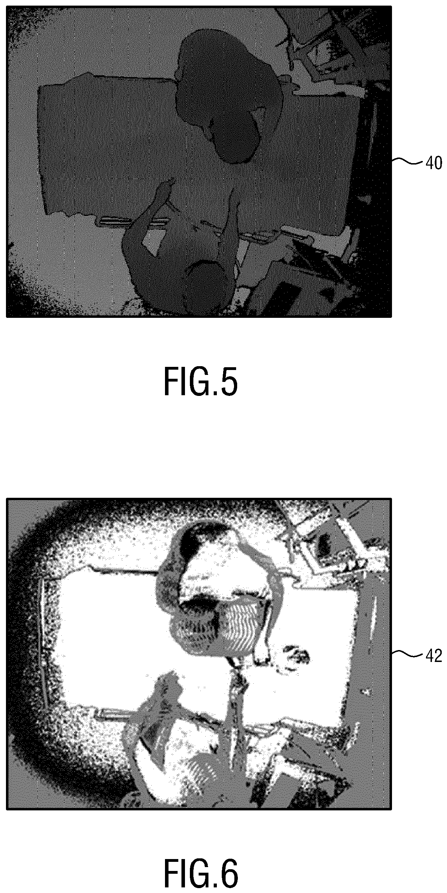

The depth camera 10 is preferably a time-of-flight 3D camera that captures depth images. In an embodiment, the camera is mounted above the patient bed so that it can obtain a top-down view of the patient. In such an example depth image 40 as shown in Fig. 5, the pixel value indicates the absolute distance between an object and the camera. In time-of-flight 3D cameras, these pixel values are computed based on reflections of the object by an emitted near-infrared light from the camera. Therefore, the pixel value can contain noise variations due to several factors. The first factor is the absorptivity or the reflectivity of the object material (e.g. bed rails - a highly reflective metallic surface). The second factor is that reflected light from two nearby objects can reach the same camera pixel. Here, a temporal variation could also occur depending on which reflected light reaches the same pixel over time. This variation is seen very frequently at object boundaries. The third factor is that the camera itself will mark a pixel as a zero value (black hole) due to either no light reaching the pixel or due to compensating for previously mentioned factors.

Based on the captured depth image, the possibility of a pixel belonging to the bed or the edge of the bed is computed using the three parameters listed below (step S10, e.g. carried out by the edge pixel confidence unit 50).

The noise variance map 42, shown as an example in Fig. 6, is computed by computing the temporal noise variations (captured by multiple depth images (a time series) over a time window) of a pixel to determine ROI boundaries. It is known that the ROI is a stable area containing less noise variations and less invalid pixels. Only the boundaries of the ROI contain noise variations. Therefore, analysis of the noise variations of a depth image helps to determine the ROI. In an embodiment, to estimate the noise variation of each pixel, the noise factors described above are modeled. This model may be a Gaussian model 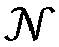 (*µ*, σ) representing a Gaussian error function (or distribution) around the true value *µ* with a standard deviation *σ* over a short time window. The variations can come from two sources: noise and motion. Due to inertia, human motion will need a longer time duration to induce pixel variances, while a short time duration, e.g., 500 ms, will mostly capture noise variances. The output of this model is shown in the noise variance map 42 depicted in Fig. 6. It can be seen that the image corners and the edges, in general, have a low confidence while stable areas, like the bed and the floor, have a high confidence. In this way enhanced edges are obtained easily of the ROI.

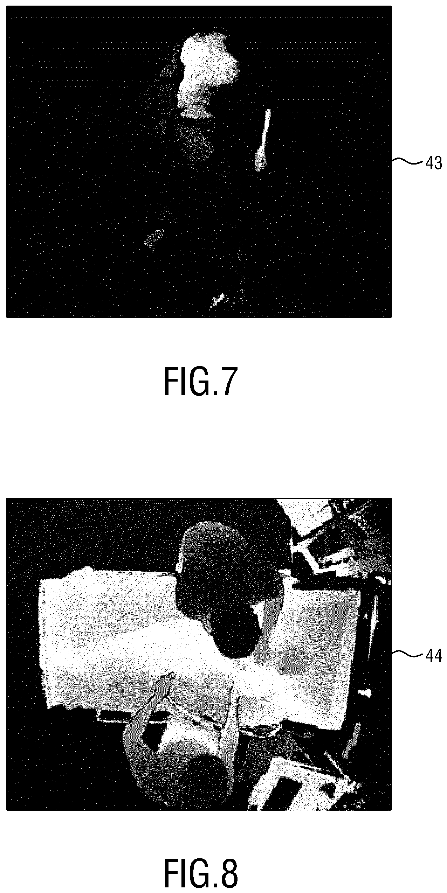

The motion confidence map 43 is shown as an example in Fig. 7. Some variations of a pixel value can also be due to motion artefacts. Due to inertia, human motion will need a longer time duration to induce pixel variances. This can be used to differentiate between pixels variations due to motion over noise. The motion confidence map 43 hence shows examples of identified motion variations. These pixels can be added to the ROI region as patient motion or can be removed from the ROI region as motion from other people (e.g., nurse) depending on their location. The motion confidence map 43 may be computed by looking at multiple depth images over a time window. This time window is larger than the time window for computing the noise variance map 42. Motion induced variations can then be captured by such a large time window.

The depth confidence map 44 is shown as an example in Fig. 8. As the pixel value indicates the distance between the object and the camera, this depth value can be used to further filter pixels in the object (e.g. bed) region. However, in practice the object can be lowered, raised, or tilted. Therefore, an adaptive filtering of the object depth value. In an embodiment the object depth is modeled with a Gaussian model / distribution to compensate for these diverse conditions of the object. This Gaussian model uses a standard mean value for the object height on initiation. After that, the model learns and adapts by using the object height of the detected region of interest in the previous iteration to filter pixels of the new object region. The depth confidence map 44 provides a visualization of these filtered depth confidence values. It can be seen that the large area of floor is marked in black and the head regions of people next to the bed are also marked in black.

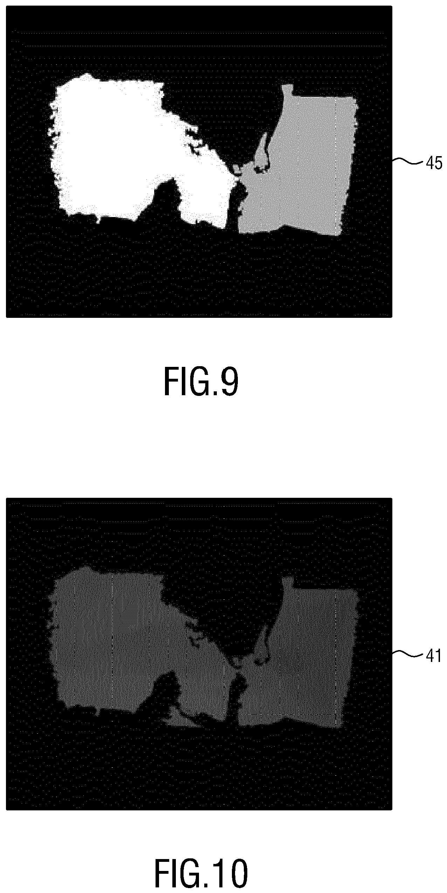

The previously computed edge pixels are then used to compute a region confidence map 45 as shown as an example in Fig. 9. The aim is to find regions that are part of the ROI. First, some region candidates are found based on contour detection and then the contour area confidences for these candidates are computed.

For contour detection, based on the three maps (noise variance, motion confidence and depth confidence), a joint confidence map is computed. Then, contour detection is applied on the binarized version of this joint confidence map. The detected contours are the candidate regions for the ROI.

Areas of the contours may indicate whether they belong to the object ROI assuming a typical area for a patient bed. However, in practice, the size of object (bed) may vary depending on the type of bed and the distance between the camera and the object. To compensate for these diffident object area conditions, a Gaussian distribution may be applied on the computed area of the contours. Then, the probability of the area belonging to the ROI is enhanced by multiplying it with the joint confidence map. This is the final contour area confidence. As can be seen in the region confidence map 45, the colors (grey values) of the two different contour regions indicate different area confidences.

The detected contours and their confidences are the candidates for object region(s). The area confidence of these contours are ranked in descending order. Then, these contour regions are combined (starting with the highest rank) at a time, and the sum of their confidences is computed at every step. The procedure of combining is stopped when the computed sum of the confidences converges. In the end, the contours that were combined are selected as the final ROI output 41 as shown in Fig. 10. In the detected ROI shown in Fig. 10 it can be seen that two contour regions are selected as the final ROI.

The present invention can be applied in the context of any type of video-based monitoring applications (such as but not limited to vital signs monitoring, delirium detection, video-actigraphy) in hospital settings (such as but not limited to ICUs, general wards, emergency rooms, waiting rooms). It finds particular application in the field of video-based actigraphy for delirium detection. Delirium detection using video-based actigraphy is promising because a camera system can observe motoric alterations of the patient. These motoric alterations are one of the core diagnostic symptoms of delirium.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for object recognition, said device comprising:
- an input unit (21) configured to obtain a depth image (40) of a scene, the depth image comprising depth information representing a distance between a depth camera and elements of the scene depicted in the depth image,
- a computation unit (22) configured:
to compute, from the depth image,
- a noise variance map (42) by computing pixel noise variances at object boundaries of one or more objects in the depth image,
- a depth confidence map (43) by filtering depth values based on their distance to the depth camera, and
- a motion confidence map (44) by filtering out variances caused by motion of a person in the scene,
to compute, from the noise variance map, the depth confidence map and the motion confidence map, one or more candidate regions (45) and their confidence in the depth image, a candidate region being a region potentially representing an object or a part of the object, and
to select the one or more candidate regions having the highest confidence as final region of interest (41) representing the object to be recognized.

2. Device as claimed in claim 1,
wherein the computation unit (22) is configured to recognize a bed as the object to be recognized.

3. Device as claimed in any one of the preceding claims,
wherein the computation unit (22) is configured to compute the noise variance map by computing pixel noise variances at boundaries of the object to be recognized and of one or more other objects occluding one or more parts of the object to be recognized in the depth image.

4. Device as claimed in any one of the preceding claims,
wherein the computation unit (22) is configured to compute the noise variance map by use of a noise model that models one or more noise factors.

5. Device as claimed in claim 4,
wherein the computation unit (22) is configured to compute the noise variance map by use of a noise model, in particular a Gaussian noise model, that models at least one noise factor selected from a group of noise factors including absorptivity or reflectivity of the material of an object, reflections of light from different objects reaching the same pixel, temporal variations depending on when a reflected light reaches the same pixel over time, and one or more pixels having a zero pixel value when no light reaches a pixel or light that would reach a pixel is compensated by other light.

6. Device as claimed in any one of the preceding claims,
wherein the computation unit (22) is configured to compute the depth confidence map by filtering out depth values of pixels lying outside a depth range assigned to the object to be recognized.

7. Device as claimed in claim 6,
wherein the computation unit (22) is configured to apply an adaptive filter that adaptively changes the depth range applied for filtering.

8. Device as claimed in claim 6,
wherein the computation unit (22) is configured to compute the depth confidence map by use of an object model, in particular a Gaussian object model, which models the depth of the object to be recognized.

9. Device as claimed in any one of the preceding claims,
wherein the computation unit (22) is configured to compute the motion confidence map by using the time duration to induce pixel variations to differentiate between pixel variations caused by motion and pixel variations caused by noise.

10. Device as claimed in any one of the preceding claims,
wherein the computation unit (22) is configured to compute the one or more candidate regions by computing a joint confidence map from the noise variance map, the depth confidence map and the motion confidence map and to apply contour detection on the joint confidence map to detect contours in the depth image, said contours indicating the one or more candidate regions.

11. Device as claimed in claim 10,
wherein the computation unit (22) is configured to compute the confidence of the one or more candidate regions by use of a Gaussian distribution on the respective candidate region and multiplying it by the joint confidence map to obtain a region confidence map and to select the one or more candidate regions having the highest confidence in the joint confidence map as final region of interest representing the object to be recognized.

12. Device as claimed in any one of the preceding claims,
wherein the computation unit (22) is configured to:
- rank the one or more candidate regions according to their confidence,
- iteratively combine candidate regions according to their rank,
- compute the sum of their confidence at every iteration,
- stop the iteration when the computed sum of the confidence converges, and
- select the candidate regions combined up to stop of the iteration as final region of interest representing the object to be recognized.

13. System for object recognition, said system comprising:
- a depth camera (10) configured to acquire a depth image of a scene, the depth image comprising depth information representing the distance between the depth camera and elements of the scene depicted in the depth image,
- a device (20) as claimed in any one of the preceding claims for object recognition based on the acquired depth image.

14. Method for object recognition, said method comprising:
- obtaining a depth image (40) of a scene, the depth image comprising depth information representing the distance between a depth camera and elements of the scene depicted in the depth image,
- computing, from the depth image,
- a noise variance map (42) by computing pixel noise variances at object boundaries of one or more objects in the depth image,
- a depth confidence map (43) by filtering depth values based on their distance to the depth camera, and
- a motion confidence map (44) by filtering out variances caused by motion of a person in the scene,
- computing, from the noise variance map, the depth confidence map and the motion confidence map, one or more candidate regions (45) and their confidence in the depth image, a candidate region being a region potentially representing an object or a part of the object, and
- selecting the one or more candidate regions having the highest confidence as final region of interest (41) representing the object to be recognized.

15. Computer program comprising program code means for causing a computer to carry out the steps of the method as claimed in claim 14 when said computer program is carried out on the computer.
